# EUROPEAN PATENT SPECIFICATION

(11) **EP 2 362 801 B1**
(45) Date of publication and mention of the grant of the patent: **02.11.2016**
(21) Application number: 09783281.0
(22) Date of filing: 22.09.2009
(51) Int. Cl.: A61N 1/375

(54) **HYPERBOLOID ELECTRICAL CONNECTOR ASSEMBLY**
ELEKTRISCHE HYPERBOLOID-VERBINDUNGSANORDNUNG
ENSEMBLE CONNECTEUR ÉLECTRIQUE HYPERBOLOÏDE

(30) Priority: 24.09.2008 WO PCT/EP2008/062762
(43) Date of publication of application: 07.09.2011
(73) Proprietor: Sorin CRM SAS, 92140 Clamart (FR)
(72) Inventor: TROOSTERS, Michel, B-1325 Dion-valmont (BE); BERTHIN, Claude, F-60127 Morienval (FR)
(74) Representative: Mellet, Valérie Martine
(86) International application number: PCT/EP2009/062256
(87) International publication number: WO 2010/034709

(56) References cited:
- EP-A- 0 587 379
- EP-A- 1 638 170
- US-A- 6 102 746
- US-A1- 2003 139 096
- US-B1- 7 347 746

## Description

### TECHNICAL FIELD

The present invention relates generally to the field of implantable medical devices. More particularly, the invention relates to an electrical connector assembly for connecting implantable medical devices, such as a pacemaker or a neurostimulator for example. However, the invention has also applicability to electrical terminals generally, irrespective of the medical implantable device to which the implantable lead has to be connected.

### DESCRIPTION OF RELATED ART

Implantable medical devices are well known in the medical field. Implantable medical devices are generally used for treating particular diseases or disorders in a patient. Examples of such implantable medical devices are: cardiac pacemakers, implantable drug infusion pumps, implantable neurostimulators, defibrillators, cochlear implants and so on.

Typically, implantable devices are subcutaneously inserted in a patient to be treated and are powered either by an internal power source, such as a primary or a secondary battery, or an external power source (that, through the skin of the patient, delivers energy to the implanted device via thin wires) or by means for transcutaneously transferring energy via inductive coupling.

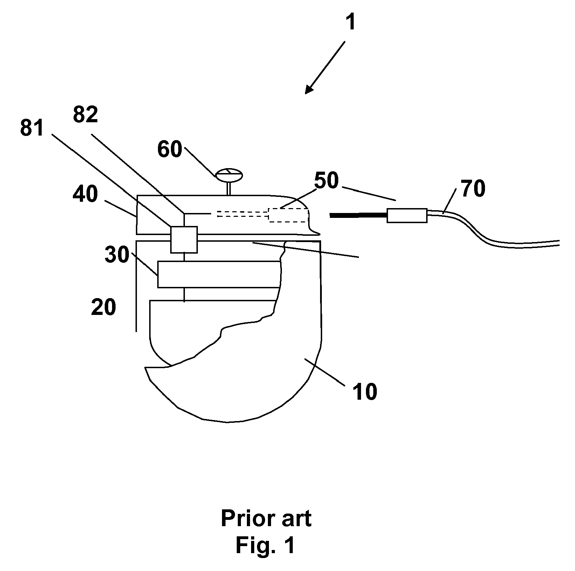

As schematically shown in **Fig.1****,** an implantable device 1, such as a pacemaker or a neurostimulator, typically comprises a battery 20 and circuitry 30 which are enclosed and sealed in a housing 10 and sends electrical signals to a specific internal part of the human body (e.g. the heart or a nerve) by means of one or more electrodes connected through an implantable lead 70. The housing 10 comprises, according to the prior art, a flat side 80 which is adapted to receive a header 40, the latter comprising a lead receiving channel 50 wherein the implantable lead 70 can be inserted. Said lead receiving channel 50 is electrically connected to circuitry 30 by means of a feedthrough connector 81 through a connection wire 82. In such a way it is possible to establish electrical connection between the implantable device 1 and the implantable lead 70 or with other implanted components such as sensors or an internal antenna for telemetry-based coupling with a second antenna of an external unit. The implantable lead 70 is firmly held within the lead receiving channel 50 by means of a retention means 60, which can be for example a set screw tightened during implantation by means of a torque wrench. Retention means 60 also provides an electrical coupling between the implantable lead 70 and the implantable device 1. In such a configuration, it is essential that housing 10 be protected against the ingress of fluids. The feedthrough connector 81 typically comprises an electrically conducting pin surrounded by insulating material, the insulating material being sealed to the housing 10. The feedthrough connector must remain fluid-tight even when a force is applied to the pin. An example of such an implantable medical device is described in document US 2008/0009912 A1**,** which describes means for connecting implantable leads 26 to an external header 22 and for connecting the header 22 to the sealed enclosure 24. The sealed enclosure 24 comprises a number of feedthrough terminal pins 74. As discussed in paragraph 27 of this document, the feedthrough terminal pins 74 are connected through crimping and/or welding. Such an implantable device presents the drawback that it is very large in size especially in case a great number of implantable leads are required and it is not, consequently, suitable for applications wherein miniature implantable medical devices are required. Moreover, when connecting header 22 to sealed enclosure 24, the crimping and/or welding is difficult or impossible to perform in operating room conditions and is an irreversible process. EP1638170 discloses an electrical connector for high current applications that is not suited for connecting implantable medical devices. In particular, such a connector is not fluid-tight against the ingress of human fluids and cannot be small in size, especially when more than 3 electrical contacts is needed.

Typically, the materials used to construct the implantable lead must be biocompatible. For providing insulation, common selections are silicone rubber, polyurethane, Teflon, etc. For the conductors, instead, common selections are stainless steel, or platinum and its alloys.

During the implantation of such medical devices within the human body, it may be necessary to establish several electrical connections for correctly installing the medical device. Such electrical connections must be sealed to protect the internal components from the human body's aggressive environment or to prevent undesired electrical signals from passing to surrounding tissue. It is clear, in fact, that any leakage of body fluids or passage of body ions into said housing 10 can result in a deterioration of the medical implantable device.

### PRIOR ART DISCUSSION

An example of an electrical connector for an implantable device is disclosed by document US 7,305,267 B2 wherein a proximal end 107 of a lead 110 is plugged into a connector module 106 in order to couple electrodes 22, 24 to an implantable medical device 100. Said connector module 106 comprises a connector bore 120 for hosting said proximal end 107 and a set-screw 127 that, in combination with a threaded connector block 129, serves as an electrical engagement means as well as a secure retention means, when fastened down on said proximal end 107. However, such a connector requires during the implantation the fastening of set-screw 127 by means of assembly tools in order to fix the implantable lead, which is very uncomfortable, difficult, time consuming, especially in operating room conditions, and may cause damages to the connector. Moreover, this connector requires an insertion force which might damage fragile parts (especially insulating parts made up of ceramic or glass) of the module 106. Furthermore, only unipolar or bipolar leads can be connected using this connector.

According to the prior art, electrical contacts between implantable leads and the contacts of an implantable device may also be realized by means of mechanical springs which are located within the connector receptacles of the header, as in the connector described for example in document US 5,730,628 B1. However, a disadvantage of such a solution consists in that these mechanical springs tend to deform permanently and therefore not to assure the necessary contact force and electrical coupling. Moreover this connector requires an insertion force which might cause damages to fragile parts (especially insulating parts of feedthrough connectors made up of ceramic or glass) of the header.

It is also known from document WO 90/02581 A1 a feedthrough connector for implantable device which comprises a barrel assembly 74 (the female connector) which is fitted within the implantable device and has a closed end 76 and an open end 78, the latter which provides a channel wherein receiving an implantable lead 64 having a first contact 66 and a second contact 68. Said first contact 66 and second contacts 68 make electrical contact with a first conductor spring 90 and a second conductor spring 96 respectively. The barrel assembly 74 comprises a first conductive portion 80 (electrically coupled with first conductor spring 90) and a second conductive portion 84 (electrically coupled with second conductor spring 96) separated by nonconductive (ceramic or glass) insulating portions 81, 83, 91, 93. The outer sides of the conductive portions 80, 84 are electrically coupled to the implantable device. No header is used in this design. However, the feedthrough connector described in this document is adapted to be used with standard bipolar pacemaker leads, such as IS-1A, IS-1 or IS-1B leads. As a consequence, this connector is not suitable for miniature implantable devices in the case where leads with multiple contacts are required, since its size would be too large and the manufacture of such a connector would be very complex. Moreover, this connector presents the drawback that the water tightness of the barrel assembly 74 is very difficult to obtain since it requires the melting and fusion of a seal portion with a conductive section. Furthermore, the insertion of the lead 64 within the barrel assembly 74 may produce on the one hand unwanted electrical contacts due to the misalignment between conductive elements (for example, the first contact pin 66 may be in electrical contact with the second spring contact 96) which may cause damages to the implantable device, and on the other hand damages to the conductive portions 80, 84 and/or the non-conductive ceramic portions 81, 83, 91, 93. Another example of a connector assembly without a header is provided by EP0587379. Pin socket 50 and conductive ring 48 of lead 14, connect to conductive pin 22 and ring terminal 24 of receptacle 10, respectively. The insertion of lead 14 in receptacles 10 requires an insertion force, in order to deform spring contacts 28 and 60, and sealing rings 64 and 70. This insertion force may damage glass seals 32, 34, 38 and ceramic insulators 30 and 36. E.g. the sealing ring 64, when in frictional contact with ceramic insulator 30, will submit glass seals 34 and 38, as well as insulators 30 and 36 to a force, and put the integrity of these elements at risk. The assembly of receptacle 10 to housing 80 through brazing or welding is also a source of weakness.

It is also known from document US 5,070,605 B1 a connector for coupling an implantable lead to an implantable device mainly comprising a connector block or header 100 in electrical contact with the implantable device 122. The connector block 100 is provided with a lumen, in which a plurality of rings is linearly arranged. The rings are so sized as to frictionally engage the implantable lead which is provided with a plurality of contacts. This plurality of rings comprises conductive rings which are in electrical contact with the implantable device through feedthrough conductive wires 128 and 130 and which engage with the contacts of the lead. Others rings of said plurality are nonconductive and act as insulators and fluid seals between the conductive rings. However, this connector is not suitable for applications wherein miniature implantable devices are to be coupled with implantable leads having several contacts, since in that case it would be necessary to increase the number of rings which would result in a header too large in size and too complex to be manufactured. Furthermore, the insertion of the lead within the connector block may produce unwanted electrical contacts, similarly to the previous prior art document, which may cause damages to the implantable device. Finally, the insertion force for inserting the lead within the connector block may cause damages to the feedthrough 124 and 126.

It is also known from document WO2008/025159 A1 a connector for implantable device which comprises a male portion 20 and a female portion 30, the latter including a protrusion 37 designed to engage a complimentary recess 27 in the male portion 20. This connector is either connected to an implantable device through cables 12, 13 or through an external header 520. However, this connector presents the drawback that it requires additional elements such as cables 12, 13 or the header 520 which are cumbersome. Furthermore, the engagement of the protrusion 37 within the recess 27 requires an insertion force which may cause damages to the It should be noticed that if the water tightness is to be obtained through other means or materials, this device would be much more complex and larger in size.

The present invention aims at providing a device that overcomes the above-discussed drawbacks of the prior art.

In particular, it is an object of the present invention to provide an electrical connector assembly which simplifies the connection process of a first implantable medical device with another implantable medical device, such as a header or an implantable lead.

Another object of the present invention is to provide an electrical connector capable of providing the mechanical and electrical connection between the implantable device and the header or implantable lead in a less complex manner and using fewer components with respect to the prior art.

More particularly, it is an object of the present invention to provide an electrical connector which is small in size, which has a high degree of reliability of the electrical contacts, and providing a high degree of reliability and water-tightness of the feedthrough connectors. Moreover, such a connector assembly should remain small in size even in case of implantable leads with several contacts.

### SUMMARY OF THE INVENTION

The invention relates to an electrical connector assembly for coupling a first implantable medical device to a second implantable medical device, said electrical connector assembly comprising a male element having one or more male contacts adapted to be electrically coupled with said first implantable medical device and a female element comprising a socket having one or more correspondent female contacts adapted to be electrically coupled with said second implantable medical device and adapted for receiving said one or more male contacts, wherein said one or more male contacts are sealed to said male element through a glass or ceramic sealing material, wherein said male element is a feedthrough connector and further wherein said female contacts of said socket comprise a contact structure comprised of a plurality of conductive elongated wires which extend along the internal surface of said female contact in a hyperboloid arrangement, thereby providing an electrical coupling between said female contact and the corresponding male contact in a plurality of points.

With preference, one or more of the following features can be used to further define the invention:
- Said conductive wires are made of a biocompatible conductive material selected from the group comprising: stainless steel, platinum, gold, or gold plated beryllium copper.
- Said socket has a diameter comprised between 2 and 8 mm.
- Said female contact has an external diameter (Dex) comprised between 0.8 and 1.2 mm.
- Said conductive wires forming said contact structure have a diameter of 100 µm.
- Said male contacts are made of a biocompatible conductive material selected from the group comprising: stainless steel, platinum, gold, or gold plated beryllium copper.

According to a seventh embodiment, the invention relates to an assembly of a first implantable medical device and a second implantable medical device comprising an electrical connector assembly according to the first aspect of the invention or to its first embodiment, wherein said male element is positioned at a surface of said first device, and said female element is positioned at a surface of said second device, said second device being a header adapted for being assembled with said first device.

According to an eighth embodiment, the invention relates to an assembly of a first implantable medical device and a second implantable medical device comprising an electrical connector assembly according to the first aspect of the invention or to its first embodiment, wherein a receptacle is provided at a surface of said first device, said male element being positioned at the bottom of said receptacle, and wherein a matching protrusion is provided at a surface of said second device, said female element being positioned at an extremity of said protrusion, said second device being an header adapted for being assembled with said first device.

According to a ninth embodiment, the invention relates to an assembly of a first implantable medical device and a second implantable medical device comprising an electrical connector assembly according to the first aspect of the invention or to its first embodiment, wherein a receptacle is provided at a surface of said first device, said male element being positioned at the bottom of said receptacle, said second device being a lead, said socket being adapted to fit into said receptacle. With preference, the assembly further comprises self-locking means capable of firmly retaining the female element within said receptacle of the male element.

Preferably, in the eighth and ninth embodiment, said female element comprises sealing means for sealing said male contacts and said female contacts from the environment when electrical coupling between the male and the female element is established.

### BRIEF DESCRIPTION OF THE DRAWING

Fig.1 is a schematic view of an implantable device such as a pacemaker, according to the prior art.
Fig.2a, 2b, and 2c are schematic views of implantable medical devices using electrical connector assemblies according to three embodiments of the present invention.
Fig.3 is a schematic representation of a hyperboloid contact structure.
Fig.4 is an end view of the connector socket of Fig.2a, 2b or 2c.
Fig.5 is a more detailed view of the electrical connector assembly of Fig.2c.
Fig.6 is a schematic sectional view of the female element of the electrical connector assembly of Fig.5.
Fig.7a and Fig.7b show schematic sectional views of the self-locking means of the female element of Fig.6.

### DETAILED DESCRIPTION OF THE INVENTION

The invention provides an electrical connector assembly for coupling a miniature implantable device to an implantable lead. The assembly comprises a male element having one or more male contact electrically coupled with said miniature implantable device and a female element comprised of a socket having one or more correspondent female contact electrically coupled with said implantable lead and capable of receiving said one or more male contact. The male element is adapted to be fitted within and integral with said miniature implantable device so that it forms a receptacle adapted to receive and fixedly maintain said female element. The male contacts are sealed to the male element through a glass or ceramic sealing material.

Each female contact of said socket comprises a contact structure comprised of a plurality of conductive elongated wires which extend along the internal surface of said female contact in a hyperboloid arrangement, whereby providing an electrical coupling between said female contact and the corresponding male contact in a plurality of points.

The conductive wires are made of a biocompatible conductive material selected from the group comprising for example: stainless steel, platinum, gold, or gold plated beryllium copper.

The electrical connector assembly may comprise self-locking means capable of fixedly retaining the female element within the receptacle of the male element.

The female element may comprise sealing means for sealing said male contacts and said female contacts from the environment when the electrical coupling between the male element and the female element is established.

The socket has a diameter of 5 mm while the female contact has a diameter of 1 mm.

The conductive wires forming the contact structure have a diameter of 100 µm and are made of a biocompatible conductive material selected from the group comprising: stainless steel, platinum, gold, or gold plated beryllium copper.

The invention also provides a miniature implantable medical device adapted to be coupled to an implantable lead. The medical implantable device comprises the male element of the electrical connector assembly of the invention. The miniature implantable device can be a pacemaker or any other implantable stimulator or electronic device.

The electrical connector assembly according to the invention may be used for establishing electrical connection between a miniature implantable device and an implantable lead.

### First embodiment

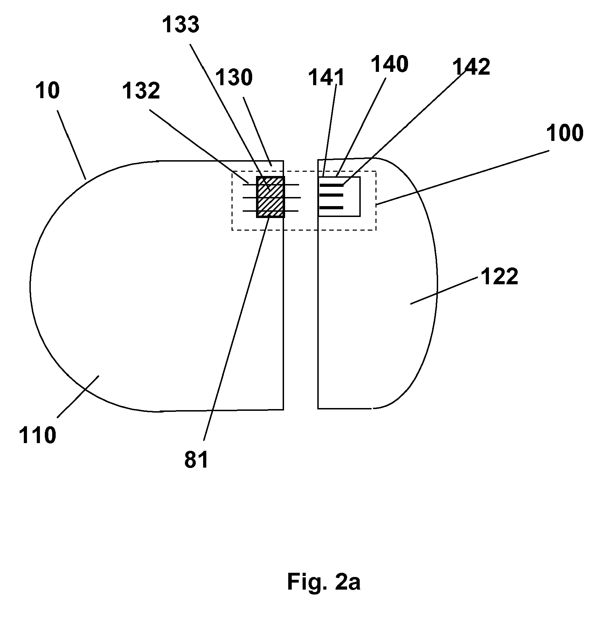

In a first embodiment of the invention represented on **Fig. 2a****,** a housing 10 includes sensitive electronic components which must be protected from body fluids of an implantable medical device 110. Electrical contacts between these electronic components and the outside world are provided by a male element 130 which is a feedthrough connector 81 comprising a plurality of feedthrough pins 132 attached to and electrically insulated from housing 10 through a ceramic or glass material 133. A header 122 comprises a female element 140 which is a socket 141 including a plurality of female contacts 142. The header 122 may provide a connection to an implantable lead or contain equipment such as an antenna. In this embodiment, the assembly of the housing 10 to header 122 is performed in a factory, under controlled clean-room conditions, including positioning means for correctly positioning header 122 in relation to housing 10. The assembly of implantable medical device 110 with header 122 is performed by gluing or any other fastening means such as screws or snap-fit. Gluing is the preferred method because it realises a watertight protection of the contact assembly 100.

### Second embodiment

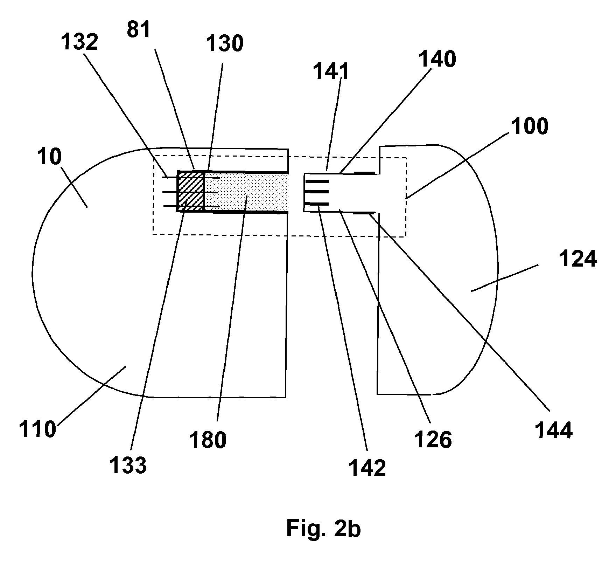

A second embodiment of the invention is represented on **Fig. 2b****,** where corresponding elements have same reference numbers as in Fig.2a. In this embodiment, the housing is provided with a receptacle 180, having a generally cylindrical shape. The male element 130 is located at the bottom of this receptacle 180. The header 124 is provided with a protrusion 126. The female element 140 is adapted at the extremity of protrusion 126, in such a way that, upon insertion of protrusion 126 into receptacle 180, the feedthrough pins 132 of male element 130 will contact corresponding female contacts 142 of socket 141. In this embodiment, sealing means 144 such as O-rings may be adapted around protrusion 126, which will ensure that no fluid can reach the contacts 132 and 142. Silicone may be used for these seals. It is to be noted that, when inserting protrusion 126 into receptacle 180, the deformation and frictional forces generated by sealing rings are not borne by the ceramic material 133 of the feedthrough connector 81.

### Third embodiment

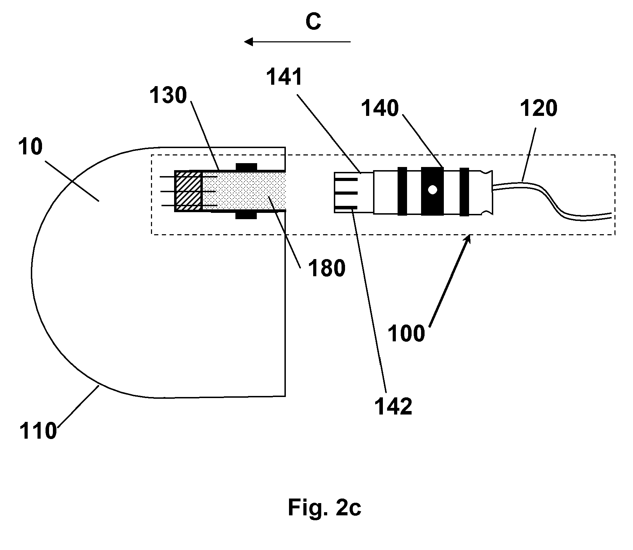

A third embodiment of the invention is represented on **Fig. 2c****,** where also corresponding elements have same reference numbers as in Fig.2a. In this embodiment, no header is used. Instead, an implantable lead, e.g. for connecting to a nerve cuff or to a heart stimulation electrode, is connected to a socket 141. The socket 141 comprises a plurality of female contacts 142, and the socket 141 is inserted directly into the receptacle 180 of the first implantable device 110. The shape of receptacle 180 and of socket 141 may be designed such that a socket fits only one way into receptacle, and that correct polarisation of the connector assembly is obtained. As in the second embodiment, one or more sealing rings may be provided for ensuring protection of the contacts. With this embodiment, the assembly of the lead to the implantable device 110 may be performed by a surgeon, in operating room condition, without using any special tool, and without risk of fracturing the ceramic material 133 of the feedthrough connector 81. With this third embodiment of the invention, more compact connector assemblies may be produced because no header is used.

### Hyperboloid contact structure

Hyperboloid electrical contacts are known in the art for having very high reliability, good resistance to vibration, as well as a long durability and are used in aerospace and automotive applications. Hyperboloid contacts are disclosed for example in documents US 4,203,647 B1, US 2002/0037674 A1, or EP 1158620 A1. However, hyperboloid electrical contacts have never been applied to solve the miniaturization requirement problem in human implantable devices. In order to obtain a reliable contact, and avoiding the risks of ceramic or glass fracture when assembling or disassembling connector assemblies, it has been found by the inventors that using hyperboloid electrical contacts in this medical application provided unexpected advantages.

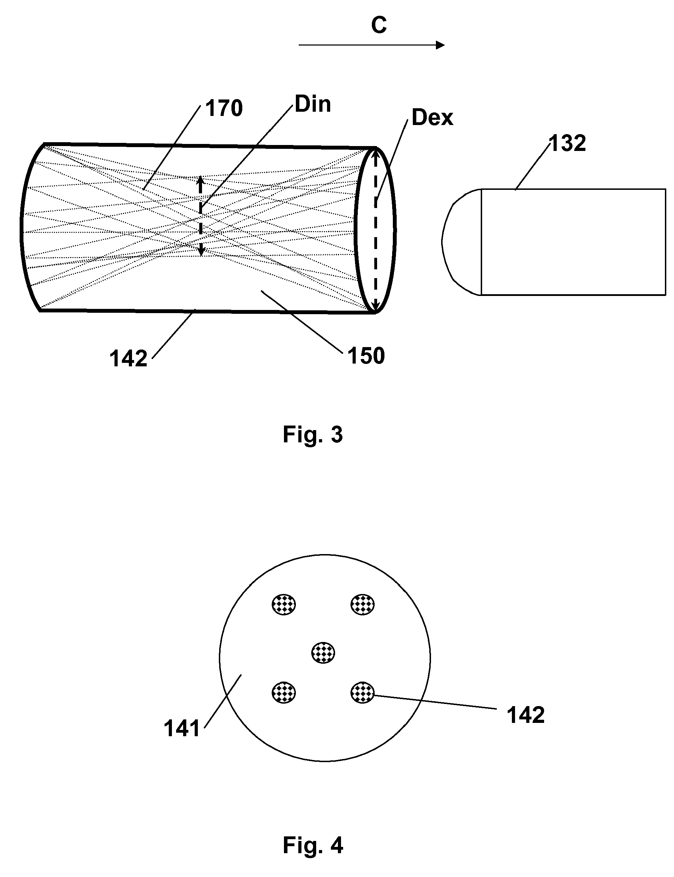

The contacting structures of all three embodiments of the invention are described by referring to **Fig. 3****.** Each female contact 142 comprises a plurality of conductive linear extended wires 170 which extend longitudinally along the internal region of said female contact 142 and whose the opposite ends are displaced with respect to each other through a small angle around the section (typically circular) of the female contact 142. This particular arrangement forms a hyperboloid structure 150 which acts as a lumen wherein the corresponding feedthrough contact pin 132 of the male element 130 is inserted. It should be noticed that the internal diameter Din of the hyperboloid structure 150 is smaller than the outer diameter Dex, whereby the female contact 142 is capable of fixedly maintaining the corresponding feedthrough contact pins 132 and guaranteeing the electrical contacts into a plurality of points therewith.

Since the use of such a hyperboloid structure 150 only requires a very low insertion force, it is possible to establish the electrical contact without causing damages to the insulating material 133 of the male element 130 neither to the connector socket 141 of the female element 140.

Said female contact 142 has a diameter of 1 mm, while the wires 170 forming the contact structure 150 have a diameter of 100 µm. Wires of the contact structure 150 as well those of the feedthrough pins 132 are made of a biocompatible metal such as stainless steel, platinum, gold, or gold plated beryllium copper. In particular the choice of gold plated beryllium copper is advantageous since this material is much more elastic than other biocompatible metals.

In view of the above, the electrical contact assembly 100 of the invention is perfectly adapted to be used for implantations of miniature medical implantable devices because of its small size as well of the fact that it is adapted to be fitted within and integral with such a miniature implantable device.

**Fig. 4** shows the connector socket 141 of the female element 140 viewed along arrow "C" of **Fig. 2c****.** Said connector socket 141 comprises five female contacts 142 and its diameter is 5 mm. However, the number and the location of the female contacts 142 may vary depending on the application. It should be noticed that the connector socket 141 has a high density of contacts and may comprises even more than ten female contacts 142, whereby it is possible to establish electrical coupling with leads having a larger number of contacts with respect to conventional bipolar leads of the prior art without increasing the dimension of the connector assembly 100. For a connector having two contacts 142, the diameter may be as small as 2mm. For a connector having twelve contacts 142, a larger diameter such as 8mm may be necessary.

### Locking mechanism

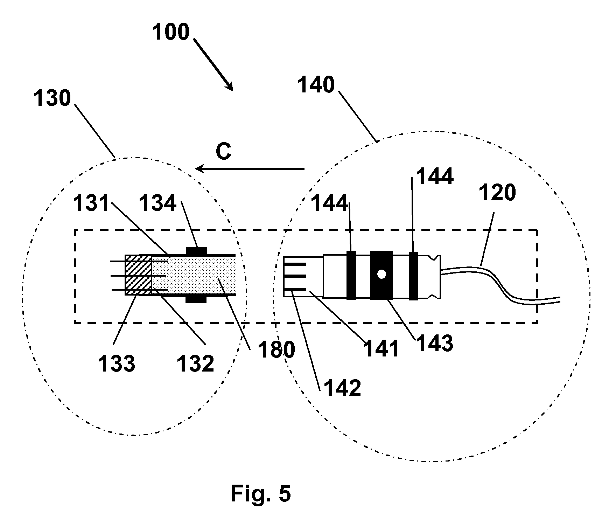

As schematically shown in **Fig. 5****,** the electrical connector assembly 100 comprises a male element 130 and a female element 140. The female element 140 is adapted to be inserted within the male element 130. The male element 130 comprises a cylindrical frame 131 which forms a receptacle 180 for receiving the female element 140 and feedthrough contact pins 132. These feedthrough contact pins 132 are maintained within the male element 130 and sealed from the internal part of the implantable device 110 by means of an insulating material 133 made up of ceramic or glass. The frame 131 further comprises a groove 134 formed inside the receptacle 180 for operating together with a self-locking means 143 of the female element 140 in order to fixedly retain the female element 140 within the male element 130, as further described. The female element 140 is electrically coupled with the implantable lead 120 and comprises a connector socket 141 with one or more female contacts 142 and has a complementary shape with respect to the cylindrical frame 131 of male element 130 so that it can be easily inserted within the male element 130 in the receptacle 180. The female element 140 further comprises sealing means 144 which consists in rings made up of silicone which are provided to tightly engage the female element 140 within the male element 130 and to seal the connector assembly 110 from the environment once the connection between the male and female elements 130, 140 has been established.

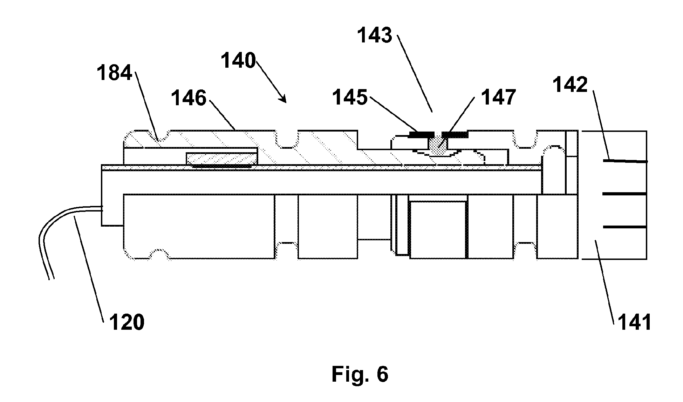

By referring to **Fig. 6****,** the female element 140 further comprises self-locking means 143 which comprises an expandable ring 145 made up of stainless steel which is wrapped around the surface of female element 140, a sliding member 146 and a rigid metallic ball 147. The expandability of said ring 145 is obtained by means of an opened section of said ring 145 which creates two separated portions of the rings 145 that are capable of radially expanding by the rigid ball 147. The ring 145 further comprises an opening for receiving a portion of the ball 147 so as to maintain the ball 147 in a fixed position with respect to the ring 145.

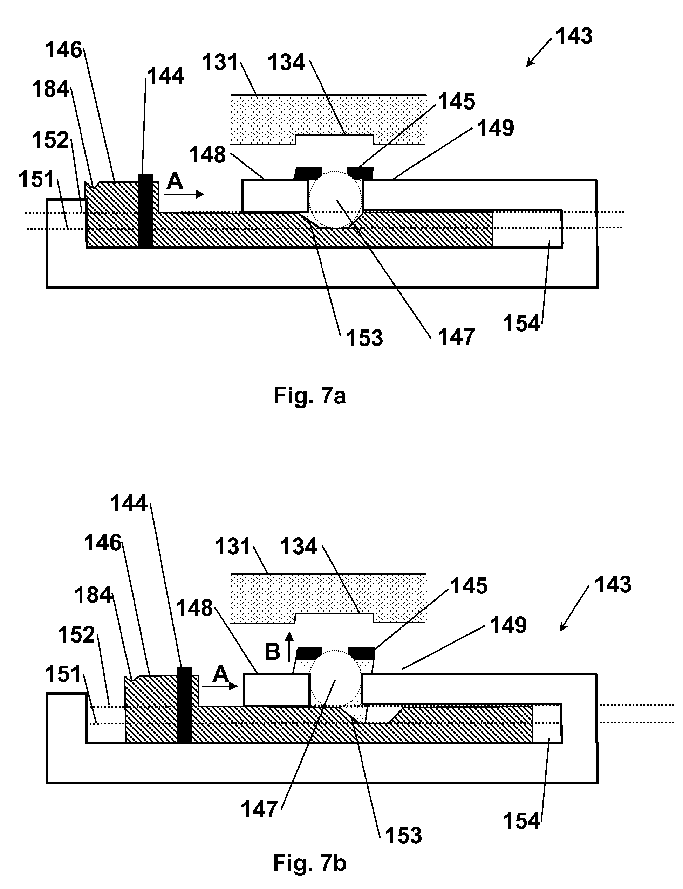

We refer now to **Fig. 7a****.** When the female element 140 is inserted within the receptacle 180 of the male element 130, it is possible to slide the sliding member 146 in the direction of arrows A within an empty portion 154 of the female element 140. When the sliding member 146 slides in the direction of arrow A, the rigid ball 147 is on the one hand maintained by elements 148, 149 of the female element 140 and by the opening of the ring 145 so that it cannot move laterally, while on the other hand it is gradually elevated in the direction of arrow B whereby passing from a lower level 151 to an upper level 152. The elevation of the rigid ball 147 is caused by the fact that when the sliding member 146 slides along the direction of arrow A, the rigid ball 147 is forced to climb up the inclined profile 153 of the sliding member 146. When the rigid ball 147 is on the lower level 151 the self-locking means 143 is accordingly in an unlocked position, while when it is on the upper level 152, the self-locking means 143 is in a locked position.

As shown in **Fig. 7b****,** when the rigid ball 147 reaches the upper level 152, it causes the two separated portions of the ring 145 to expand and consequently enter the corresponding groove 134 of the male element 130 so that the female element 140 is mechanically blocked within the receptacle 180 of the male element 130. When the self-locking means is in locked position, the sliding member 146 is firmly kept in place due to the adherence of sealing means 144 against the internal surface of the receptacle 180 so as to prevent the sliding member 146 from freely sliding.

The removal of the female element 140 from the male element 130 can be easily performed by means of a surgical pliers which allows gripping a cavity 184 of the sliding member 146 and sliding it in the reverse direction of arrow A so as the ball 147 gradually passes from the upper level 152 to the lower level 151 (the rigid ball 147 goes down the inclined profile 153 of the sliding member 146). As a consequence, the ring 145 returns to the position shown in **Fig. 7a** and the female element 140 can easily exit the male element 130.

The self-locking means 143 is extremely resistant to vibration and can resist to traction forces higher than 10 N, as verified by the Applicant. Moreover, the insertion force as well as the extraction force (when the self-locking means 143 is in an unlocked position) is less than 14 N as required by IS-1 regulation. The high reliability of the self-locking means 143 therefore prevents all unwanted disconnections between the female element 140 and the male element 130 of the connector assembly 100 and requires, by contrast, very low insertion/extraction force.

The terms and descriptions used herein are set forth by way of illustration only and are not meant as limitations. Those skilled in the art will recognize that many variations are possible within the scope of the invention as defined in the following claims. As a consequence, all modifications and alterations will occur to others upon reading and understanding the previous description of the invention. In particular, dimensions, materials, and other parameters, given in the above description may vary depending on the needs of the application. More specifically, a first medical device may be connected to one or more second medical devices through same or different combinations of the connector assembly of the invention.

## Claims

1. An electrical connector assembly (100) for coupling a first implantable medical device (110) to a second implantable medical device (120; 122; 124), said electrical connector assembly (100) comprising a male element (130) having one or more male contacts (132) adapted to be electrically coupled with said first implantable medical device (110) and a female element (140) comprising a socket (141) having one or more correspondent female contacts (142) adapted to be electrically coupled with said second implantable medical device (120; 122; 124) and adapted for receiving said one or more male contacts (132), **characterised in that** said one or more male contacts (132) are sealed to said male element (130) through a glass or ceramic sealing material, **in that** said male element (130) is a feedthrough connector, **in that** said male contacts (132) are feedthrough pins, **and in that** said female contacts (142) of said socket (141) comprise a contact structure (150) comprised of a plurality of conductive elongated wires (170) which extend along the internal surface of said female contact (142) in a hyperboloid arrangement, thereby providing an electrical coupling between said female contact (142) and the corresponding male contact (132) in a plurality of points.

2. The electrical connector assembly (100) according to claim 1 **characterised in that** said conductive wires (170) are made of a biocompatible conductive material selected from the group comprising: stainless steel, platinum, gold, or gold plated beryllium copper.

3. The electrical connector assembly (100) according to any of previous claims **characterized in that** said socket (141) has a diameter comprised between 2 and 8 mm.

4. The electrical connector assembly (100) according to any of previous claims **characterized in that** said female contact (142) has an external diameter (Dex) comprised between 0,8 and 1,2 mm.

5. The electrical connector assembly (100) according to any of preceding claims **characterised in that** said conductive wires (170) forming said contact structure (150) have a diameter of 100 µm.

6. The electrical connector assembly (100) according to any of previous claims wherein said male contacts (132) are made of a biocompatible conductive material selected from the group comprising: stainless steel, platinum, gold, or gold plated beryllium copper.

7. Assembly of a first implantable medical device (110) and a second implantable medical device (120; 122; 124) comprising an electrical connector assembly (100) according to claim 1 or 2, **characterized in that** said male element (130) is positioned at a surface of said first device (110), and said female element is positioned at a surface of said second device (120; 122; 124), said second device being a header (122) adapted for being assembled with said first device (110).

8. Assembly of a first implantable medical device (110) and a second implantable medical device (120; 122; 124) comprising an electrical connector assembly (100) according to claim 1 or 2, **characterised in that** a receptacle (180) is provided at a surface of said first device (110), said male element (130) being positioned at the bottom of said receptacle (180), and **in that** a matching protrusion (126) is provided at a surface of said second device (120; 122; 124), said female element being positioned at an extremity of said protrusion (126), said second device being a header (124) adapted for being assembled with said first device (110).

9. Assembly of a first implantable medical device (110) and a second implantable medical device (120; 122; 124) comprising an electrical connector assembly (100) according to claim 1 or 2, **characterised in that** a receptacle (180) is provided at a surface of said first device (110) said male element being positioned at the bottom of said receptacle (180), said second device (120; 122; 124) being a lead (120), said socket (141) being adapted to fit into said receptacle (180).

10. Assembly according to claim 9 further comprising self-locking means (134; 143) capable of firmly retaining the female element (140) within said receptacle (180) of the male element (130).

11. Assembly according to any of claims 8 to 10 **characterised in that** said female element (140) comprises sealing means (144) for sealing said male contacts (132) and said female contacts (142) from the environment when the electrical coupling between the male element (130) and the female element (140) is established.

## Patentansprüche

1. Elektrische Anschlussgruppe (100) zum Koppeln einer ersten implantierbaren medizinischen Vorrichtung (110) an eine zweite implantierbare medizinische Vorrichtung (120; 122; 124), wobei die genannte elektrische Anschlussgruppe (100) ein männliches Element (130) mit einem oder mehreren männlichen Kontakt(en) (132) umfasst, die angepasst sind, um elektrisch mit der genannten ersten implantierbaren medizinischen Vorrichtung (110) gekoppelt zu werden, und ein weibliches Element (140), das eine Fassung (141) umfasst, die einen oder mehrere entsprechende weibliche Kontakt(e) (142) hat, die angepasst sind, um elektrisch mit der genannten zweiten implantierbaren medizinischen Vorrichtung (120; 122; 124) gekoppelt zu werden und angepasst sind, um den genannten einen oder mehrere männliche Kontakt(e) (132) zu empfangen, **dadurch gekennzeichnet, dass** der / die genannte(n) eine oder mehrere Kontakt(e) (132) an dem genannten männlichen Element (130) durch ein Glas- oder Keramik-Versiegelungsmaterial versiegte ist / sind, dass das genannte männliche Element (130) ein Durchkontaktierungsanschluss ist, dass die genannten männlichen Kontakte (132) Durchkontaktierungs-Pins sind **und dass** die genannten weiblichen Kontakte (142) der genannten Fassung (141) eine Kontaktstruktur (150) umfassen, die aus einer Vielzahl von leitenden, länglichen Drähten (170) gebildet ist, die sich entlang der internen Oberfläche des genannten weiblichen Kontakts (142) in einer hyperboloiden Anordnung erstrecken und dabei eine elektrische Kopplung zwischen dem genannten weiblichen Kontakt (142) und dem entsprechenden männlichen Kontakt (132) in einer Vielzahl von Punkten bereitstellen.

2. Elektrische Anschlussgruppe (100) gemäß Anspruch 1, **dadurch gekennzeichnet, dass** die genannten leitenden Drähte (170) aus einem biokompatiblen leitenden Material hergestellt sind, das aus der Gruppe ausgewählt ist, umfassend: Edelstahl, Platin, Gold oder mit Gold plattiertes Beryllium-Kupfer.

3. Elektrische Anschlussgruppe (100) gemäß irgendeinem der voranstehenden Ansprüche, **dadurch gekennzeichnet, dass** die genannte Fassung (141) einen zwischen 2 und 8 mm inbegriffenen Durchmesser hat.

4. Elektrische Anschlussgruppe (100) gemäß irgendeinem der voranstehenden Ansprüche, **dadurch gekennzeichnet, dass** der genannte weibliche Kontakt (142) einen zwischen 0,8 und 1,2 mm inbegriffenen Durchmesser (Dex) hat.

5. Elektrische Anschlussgruppe (100) gemäß irgendeinem der voranstehenden Ansprüche, **dadurch gekennzeichnet, dass** die genannten leitenden Drähte (170), die die genannte Kontaktstruktur (150) bilden, einen Durchmesser von 100 µm haben.

6. Elektrische Anschlussgruppe (100) gemäß einem der voranstehenden Ansprüche, bei der die genannten männlichen Kontakte (132) aus einem biokompatiblen leitenden Material hergestellt sind, das ausgewählt ist aus der Gruppe, umfassend: Edelstahl, Platin, Gold oder mit Gold plattiertes Beryllium-Kupfer.

7. Gruppe einer ersten implantierbaren medizinischen Vorrichtung (110) und einer zweiten implantierbaren medizinischen Vorrichtung (120; 122; 124), die eine elektrische Anschlussgruppe (100) gemäß Anspruch 1 oder 2 umfasst, **dadurch gekennzeichnet, dass** das genannte männliche Element (130) an einer Oberfläche der genannten ersten Vorrichtung (110) positioniert ist und das genannte weibliche Element an einer Oberfläche der genannten zweiten Vorrichtung (120; 122; 124) positioniert ist, wobei die genannte zweite Vorrichtung ein Verteilerstück (122) ist, das angepasst ist, um mit der genannten ersten Vorrichtung (110) zusammengesetzt zu werden.

8. Gruppe aus einer ersten implantierbaren medizinischen Vorrichtung (110) und einer zweiten implantierbaren medizinischen Vorrichtung (120; 122; 124), die eine elektrische Anschlussgruppe (100) gemäß Anspruch 1 oder 2 umfasst, **dadurch gekennzeichnet, dass** eine Steckhülse (180) an einer Oberfläche der genannten ersten Vorrichtung (110) bereitgestellt ist, wobei das männliche Element (130) am Boden der genannten Steckhülse (180) positioniert ist, und dass ein passender Vorsprung (126) an einer Oberfläche der genannten zweiten Vorrichtung (120; 122; 124) bereitgestellt ist, wobei das genannte weibliche Element an einem Ende des genannten Vorsprungs (126) positioniert ist, wobei die genannte zweite Vorrichtung ein Verteilerstück (124) ist, das angepasst ist, um mit der genannten ersten Vorrichtung (110) zusammengesetzt zu werden.

9. Gruppe aus einer ersten implantierbaren medizinischen Vorrichtung (110) und einer zweiten implantierbaren medizinischen Vorrichtung (120; 122; 124), die eine elektrische Anschlussgruppe (100) gemäß Anspruch 1 oder 2 umfasst, **dadurch gekennzeichnet, dass** eine Steckhülse (180) an einer Oberfläche der genannten ersten Vorrichtung (110) bereitgestellt ist, wobei das genannte männliche Element am Boden der genannten Steckhülse (180) positioniert ist, wobei die zweite Vorrichtung (120; 122; 124) eine Führung (120) ist, wobei die Fassung (141) angepasst ist, um sich in die genannte Steckhülse (180) einzupassen.

10. Gruppe gemäß Anspruch 9, weiterhin umfassend ein selbstverriegelndes Mittel (134; 143), das geeignet ist, das weibliche Element (140) innerhalb der Steckhülse (180) des männlichen Elements (130) fest zurückzuhalten.

11. Gruppe gemäß irgendeinem der Ansprüche 8 bis 10, **dadurch gekennzeichnet, dass** das genannte weibliche Element (140) Versiegelungsmittel (144) für die Versiegelung der genannten männlichen Kontakte (132) und der genannten weiblichen Kontakte (142) gegenüber der Umwelt umfasst, wenn die elektrische Kopplung zwischen dem männlichen Element (130) und dem weiblichen Element (140) hergestellt ist.

## Revendications

1. Ensemble de connecteur électrique (100) pour coupler un premier dispositif médical implantable (110) à un second dispositif médical implantable (120; 122; 124), ledit ensemble de connecteur électrique (100) comprenant un élément mâle (130) comprenant un ou plusieurs contact(s) mâle(s) (132) apte(s) à être couplé(s) électriquement audit premier dispositif médical implantable (110), et un élément femelle (140) comprenant une douille (141) comprenant un ou plusieurs contact(s) femelle(s) correspondant(s) (142) apte(s) à être couplé(s) électriquement audit second dispositif médical implantable (120; 122; 124) et apte(s) à recevoir ledit/lesdits un ou plusieurs contact(s) mâle(s) (132),
**caractérisé en ce que** ledit/lesdits un ou plusieurs contact(s) mâle(s) (132) est (sont) scellé(s) sur ledit élément mâle (130) par l'intermédiaire d'un matériau de verre ou de céramique, **en ce que** ledit élément mâle (130) est un connecteur de traversée, **en ce que** lesdits contacts mâles (132) sont des broches de traversée, et **en ce que** lesdits contacts femelles (142) de ladite douille (141) présentent une structure de contact (150) constituée d'une pluralité de fils conducteurs allongés (170) qui s'étendent le long de la surface intérieure dudit contact femelle (142) dans un agencement hyperboloïde, établissant de ce fait un couplage électrique entre ledit contact femelle (142) et le contact mâle correspondant (132) en une pluralité de points.

2. Ensemble de connecteur électrique (100) selon la revendication 1, **caractérisé en ce que** lesdits fils conducteurs (170) sont constitués d'un matériau conducteur biocompatible sélectionné dans le groupe comprenant l'acier inoxydable, le platine, l'or ou le cuivre au béryllium plaqué or.

3. Ensemble de connecteur électrique (100) selon l'une quelconque des revendications précédentes, **caractérisé en ce que** ladite douille (141) présente un diamètre compris entre 2 mm et 8 mm.

4. Ensemble de connecteur électrique (100) selon l'une quelconque des revendications précédentes, **caractérisé en ce que** ledit contact femelle (142) présente un diamètre externe (Dex) compris entre 0,8 mm et 1,2 mm.

5. Ensemble de connecteur électrique (100) selon l'une quelconque des revendications précédentes, **caractérisé en ce que** lesdits fils conducteurs (170) qui forment ladite structure de contact (150) présentent un diamètre de 100 µm.

6. Ensemble de connecteur électrique (100) selon l'une quelconque des revendications précédentes, dans lequel lesdits contacts mâles (132) sont constitués d'un matériau conducteur biocompatible sélectionné dans le groupe comprenant l'acier inoxydable, le platine, l'or ou le cuivre au béryllium plaqué or.

7. Assemblage d'un premier dispositif médical implantable (110) et d'un second dispositif médical implantable (120; 122; 124) comprenant un ensemble de connecteur électrique (100) selon la revendication 1 ou 2, **caractérisé en ce que** ledit élément mâle (130) est positionné à une surface dudit premier dispositif (110), et ledit élément femelle est positionné à une surface dudit second dispositif (120; 122; 124), ledit second dispositif étant une embase (122) apte à être assemblée avec ledit dispositif (110).

8. Assemblage d'un premier dispositif médical implantable (110) et d'un second dispositif médical implantable (120; 122; 124) comprenant un ensemble de connecteur électrique (100) selon la revendication 1 ou 2, **caractérisé en ce qu'**un réceptacle (180) est prévu à une surface dudit premier dispositif (110), ledit élément mâle (130) étant positionné au fond dudit réceptacle (180), et **en ce qu'**une saillie correspondante (126) est prévue à une surface dudit second dispositif (120; 122; 124), ledit élément femelle étant positionné à une extrémité de ladite saillie (126), ledit second dispositif étant une embase (124) apte à être assemblée avec ledit premier dispositif (110).

9. Ensemble d'un premier dispositif médical implantable (110) et d'un second dispositif médical implantable (120; 122; 124) comprenant un ensemble de connecteur électrique (100) selon la revendication 1 ou 2, **caractérisé en ce qu'**un réceptacle (180) est prévu à une surface dudit dispositif (110), ledit élément mâle étant positionné au fond dudit réceptacle (180), ledit second dispositif (120; 122; 124) étant un fil conducteur (120), ladite douille (141) étant apte à s'agencer dans ledit réceptacle (180).

10. Ensemble selon la revendication 9, comprenant en outre des moyens d'auto-verrouillage (134; 143) capables de retenir fermement l'élément femelle (140) à l'intérieur dudit réceptacle (180) de l'élément mâle (130).

11. Ensemble selon l'une quelconque des revendications 8 à 10, **caractérisé en ce que** ledit élément femelle (140) comprend des moyens d'étanchéité (144) pour isoler lesdits contacts mâles (132) et lesdits contacts femelles (142) de l'environnement lorsque le couplage électrique entre l'élément mâle (130) et l'élément femelle (140) est établi.
